# NEW EUROPEAN PATENT SPECIFICATION

(11) **EP 0 253 607 B2**
(45) Date of publication and mention of the opposition decision: **23.05.2001**
(45) Mention of the grant of the patent: 29.04.1992
(21) Application number: 87306174.1
(22) Date of filing: 13.07.1987
(51) Int. Cl.: A61K 31/565, A61K 31/567, A61K 31/57, A61K 31/569

(54) **Combination dosage form for premenopausal women**
Kombinations-Dosierform für Frauen in der Prä-Menopause
Forme de dosage combinée pour des femmes pré-ménopausiques

(30) Priority: 15.07.1986 US 885971
(43) Date of publication of application: 20.01.1988
(73) Proprietor: AMERICAN HOME PRODUCTS CORPORATION, Madison, New Jersey 07940-0874 (US)
(72) Inventor: Upton, Gertrude Virginia, Radnor Pennsylvania (US)
(74) Representative: Brown, Keith John Symons

(56) References cited:
- EP-A- 0 136 011
- EP-A- 0 235 090
- US-A- 3 669 502
- US-A- 3 939 264
- US-A- 3 957 982
- UNLISTED DRUGS, vol. 26, no. 10, October 1974, page 162q, Chatham, New Jersey, US; "Sophia-A"
- UNLISTED DRUGS, vol. 27, no. 9, September 1975, page 145d, Chatham, New Jersey, US; "Medicon"
- UNLISTED DRUGS, vol. 25, no. 10, October 1973, page 160a, Chatham, New Jersey, US; "Microgynon"
- UNLISTED DRUGS, vol. 27, no. 12, December 1975, page 194g, Chatham, New Jersey, US; "Menophase"
- UNLISTED DRUGS, vol. 34, no. 2, February 1982, page 29f, Chatham, New Jersey, US; "Trionetta"
- UNLISTED DRUGS, vol. 26, no. 11, November 1974, page 170b, Chatham, New Jersey, US; " WL-20"

## Description

The subject invention provides hormonal replacement therapy and contraceptive protection for the pre-menopausal woman in need thereof. Such therapy and contraceptive protection is provided by a combination dosage form of the invention which comprises a low dose of an estrogen combined with a very low dose of a progestogen. A preferred combination dosage form of the invention comprises 0.75-1.50 mg. of 17β-estradiol and 0.035-0.085 mg. of levonorgestrel. The combination dosage form of the invention is administered for the first 23-26 days of the menstrual cycle, followed by 2-5 pill-free or blank pill days, for a total of 28 days in the administration cycle. The preferred administration cycle is 24 days of the combination dosage form and 4 days of no dosage form.

Pre-menopause is defined as the time around 40 years of age when a woman can reasonably be said to be approaching menopause (the last menstrual period) or the time when a woman feels the approach of menopause by experiencing pre-menopausal irregularities in her menstrual cycle or other hypoestrogenic symptoms.

The woman over forty in is a transitional period in which her hormone levels are waning; she still ovulates and yet she experiences many of the symptoms of the hypoestrogenic woman, insomnia, hot flushes, irritability, etc. The fact that these women are still menstruating has led to the uninformed attitude that her complaints are psychosomatic in origin.

The climacteric is marked by many changes due to the natural aging process; all of which are modified by individual life-styles. Both natural and surgical menopause appear to be associated with adverse changes in metabolic parameters and in hormone levels. For example, the metabolic change in the blood lipid profile of the post-menopausal woman may lead to the development of athersclerosis, hypertension and coronary heart disease. See Notelovitz M, Graig SK, Rappaport V, et al; "Menopausal status associated with increased inhibition of blood coagulation," Am J Obstet Gynecol 141:149, (1981); Notelovitz M, Greig HBW, "Natural estrogen and anti-thrombin III activity in postmenopausal women," J Reprod Med 16:87 (1976); Nielsen FH, Honore E, Kristoffersen K, et al, "Changes in serum lipids during treatment with norgestrel, estradiol-valerate and cycloprogynon.^{R}. Acta Obstet Gynecol Scand 56:367 (1977) and Paterson MEL, Sturdee DW, Moore B, "The effect of various regimens of hormone therapy on serum cholesterol and triglyceride concentrations in postmenopausal women," Br J Obstet Gynecol 87:552 (1980). Adverse changes in hormonal levels of the post-menopausal woman are associated with endometrial and breast cancer and with osteoporosis. See Gambrell RD Jr, Bagnell CA, Greenblatt RB, "Role of estrogens and progesterone in the etiology and prevention of endometrial cancer: Review, Am J Obstet Gynecol 146:696 (1983); Gambrell RD Jr, "The prevention of endometrial cancer in postmenopausal women with progestogen,". Maturitas 1:107 (1978); and Nachtigall LE, Nachtigall RH, Nachtigall RD, et al, "Estrogen replacement therapy: I. A 10 year prospective study in the relationship to osteoporosis," Obstet Gynecol 53:277, (1979).

The years after 40 witness an ever increasing number of anovulatory cycles, leaving a woman still menstruating but exposed to variable periods of unopposed estrogen. It is impossible to predict which cycles will be ovulatory or anovulatory because of the absence of any consistent pattern. Thus, the pre-menopausal woman also needs constant contraceptive protection. If one considers the mortality rate in the aging women due to late child birth, this contraceptive need becomes of paramount importance. Therefore, in consideration of the appropriate hormone therapy for the pre-menopausal woman, attention must be focused on the effects of such therapy on metabolic parameters as well as on reproductive target organs. In the pre-menopausal woman it is necessary that such therapy also be contraceptive.

In the first aspect the invention provides the use of a composition comprising an estrogen selected from
0.75-1.50 mg of 17β-estradiol,
0.012-0.025 mg of ethinyl estradiol, and
0.025-0.050 mg of mestranol ;
and a progestogen selected from :
0.035-0.085 mg of levonorgestrel,
0.015-0.060 mg of gestodene,
0.035-0.085 mg of desogestrel,
0.035-0.085 mg of 3-ketodesogestrel, and
0.10-0.30 mg of norethindrone,
for the manufacture of a dosage form for providing hormonal replacement therapy and contraception for a pre-menopausal woman by administration of the dosage form for 23 to 26 days, beginning at day one of the menstrual cycle, followed by 2 to 5 pill- free or blank pill days, for a total of 28 days in the administration cycle.

A second aspect of the invention provides a pack for providing hormonal replacement therapy and contraception for a pre-menopausal woman which pack comprises 23 to 26 dosage forms each comprising an estrogen selected from :
(a) 0.75-1.50 mg of 17β-estradiol,
   0.012-0.025 mg of ethinyl estradiol, and
   0.025-0.050 mg of mestranol ;
   and a progestogen selected from
   0.035-0.085 mg of levonorgestrel,
   0.015-0.060 mg of gestodene,
   0.035-0.085 mg of desogestrel,
   0.035-0.085 mg of 3-ketodesogestrel, and
   0.10-0.30 mg of norethindrone, and
(b) 2 to 5 blank pills or other indications to indicate that the daily administration of the 23 to 26 dosage forms should be followed by 2 to 5 pill-free or blank pill days, for a total of 28 days in the administration cycle.

For both aspects of the invention the preferred estrogen is 17β-estradiol and the preferred progestogen is levonorgestrel. Gestodene is also a particularly preferred progestogen. A particularly preferred combination dosage form for both aspects of the invention is a combination in which the estrogen is in a dose of 1 mg of 17β-estradiol or an equivalent dose of ethinyl estradiol or mestranol and the progestogen is in a dose of 0.050 mg of levonorgestrel or an equivalent dose of gestodene, desogestrel or 3-ketodesogestrel. A further particularly preferred combination dosage form for both aspects of the invention is a combination in which the estrogen is in a dose of 1 mg of 17β-estradiol or an equivalent dose of ethinyl estradiol or mestranol and the progestogen is in a dose of 0.075 mg of levonorgestrel or an equivalent dose of gestodene, desogestrel or 3-ketodesogestrel. A preferred course of administration for both aspects of the invention is administration of the combination dosage form of the invention for the first 24 days of the menstrual cycle and no dosage form (i.e. pill-free) or a blank dosage form for the last 4 days of the menstrual cycle. A further preferred course of administration for all aspects of the invention is administration of the combination dosage form for the first 23 days of the menstrual cycle and no dosage form (i.e. pill-free) or a blank dosage form for the last 5 days of the menstrual cycle. The preferred doses equivalent to 1 mg of 17β-estradiol are, approximately: ethinyl estradiol 0.015 mg and mestranol 0.030 mg. The preferred doses equivalent to 0.050 mg of levonorgestrel are approximately : gestodene 0.035 mg, desogestrel and 3-ketodesogestrel 0.050 mg, and norethindrone 0.175 mg. The preferred doses equivalent to 0.075 mg of levonorgerel are, approximately: gestodene 0.052 mg, desogestrel and 3-ketodesogestrel 0.075 mg, and norethindrone 0.25 mg. Such equivalent doses may vary depending upon the physiological effect desired and the assay method used.

For all aspects of the invention, the preferred cycle of administration is administration of the combination dosage form for the first 24 days of the menstrual cycle and administration of no dosage form or a blank dosage form for the last 4 days of the menstrual cycle. Or, administration of the combination dosage form for the first 23 days of the menstrual cycle and no dosage form or a blank dosage form for the last 5 days is also preferred. A preferred dose of ethinyl estradiol equivalent to the preferred dose of 1 mg of 17β-estradiol is 0.015 mg. The equivalent preferred dose of mestranol is 0.030 mg. The preferred equivalent doses of desogestrel and 3-ketodesogestrel which are equivalent to the preferred doses of levonorgestrel, namely, 0.050 mg and 0.075 mg are also 0.050 and 0.075 mg. The equivalent preferred doses of gestodene to 0.050 mg and 0.075 mg of levonorgestrel are 0.035 mg and 0.052 mg.

It is to be understood that in this specification and the accompanying claims norgestrel may be used in place of levonorgestrel, but at twice the stated dose of levonorgestrel. The accompanying claims should be construed accordingly. Norgestrel is a racemic compound while levonorgestrel is one of the optically active isomers. Levonorgestrel is particularly preferred.

The progestogen levonorgestrel is well known and has been marketed in oral contraceptive formulations (at doses of 0.15 mg, and higher) for many years. Its chemical name is (-)-13-ethyl-17-hydroxy-18,19-dinorpregn-4-en-20-yn-3-one. Norgestrel's common name is 17α-ethynyl-18-homo-19-nortestosterone. Gestodene, desogestrel, and 3-ketodesogestrel are newer progestogens in various stages of clinical development and use. The new compound, gestodene, differs from norgestrel by a double bond in the 15 position and is progestationally active per se, whereas desogestrel is believed to be inactive as the parent molecule and is thought to undergo two metabolic steps for progestational activity. Desogestrel is believed to be metabolized first to the biologically active 3β-hydroxydesogestrel with estrogenic/androgenic activity and then to 3-ketodesogestrel, which has progestogenic activity; it differs from norgestrel by a methylene group at position 11. Norethindrone's chemical name is 17-hydroxy-19-norpregn-4-en-20-yn-3-one. It is also known as 19-norethisterone or norethisterone. Norethindrone acetate may be used in place of norethindrone, and hence the term "norethindrone" in the accompanying claims is to be understood as referring to either the free alcohol or its acetate.

17β-estradiol is the most potent naturally occurring estrogen in mammals. Its chemical name is estra-1,3,5(10)-triene-3,17-diol. 17β-estradiol (or β-estradiol) is its common name. Ethinyl estradiol and mestranol are both synthetic estrogens which have an ethinyl group at the 17 position of the estradiol ring structure. Mestranol additionally has a methoxy group rather than a hydroxy group at the 3 position of the estradiol ring structure. Ethinyl estradiol and mestranol are used in oral contraceptive formulations. The composition of such marketed oral contraceptives is shown in Table 15-2 on page 454 in Chapter 15 of "Fertility Control and its Complications" by Bruce R. Carr and James E. Griffin in Williams Textbook of Endocrinology, seventh edition, (Jean D. Wilson M.D. and Daniel W. Foster M.D. (W.B. Saunders Company, Philadelphia, 1985).

An example of a pack which may be used in the second aspect of the invention or for providing the dosage forms for use by the patient in other aspects of the invention is a blister pack type product as is commonly used with oral contraceptive products. Such product would normally comprise the appropriate number of dosage tablets in a sealed blister pack in a cardboard, paperboard or plastic sleeve with a protective cover or box. Each combination dosage tablet blister container may be numbered or otherwise marked for the first 23-26 days of the menstrual cycle, [as, for example, prescribed by the patient's physician]. The remaining 2-5 (pill-free) days of the 28 day administration cycle would contain blank-pills or unfilled blisters or other marking devices to assist the patient in following the prescribed administration cycle. The combination estrogen and progestogen dosage form of the invention is preferably provided as a tablet, caplet or capsule in a manner known in the art. Similarly a blank pill is preferably a tablet, caplet or capsule containing no active hormonal agents. Other oral or parenteral dosage preparations or packages may be provided as known in the art.

## Claims (Claims for the following Contracting State(s): BE, CH, DE, FR, IT, LI, LU, NL, SE)

1. The use of a composition comprising an estrogen selected from
0.75-1.50 mg of 17β-estradiol,
0.012-0.025 mg of ethinyl estradiol, and
0.025-0.050 mg of mestranol ;
and a progestogen selected from :
0.035-0.085 mg of levonorgestrel,
0.015-0.060 mg of gestodene,
0.035-0.085 mg of desogestrel,
0.035-0.085 mg of 3-ketodesogestrel, and
0.10-0.30 mg of norethindrone,
for the manufacture of a dosage form for providing hormonal replacement therapy and contraception for a pre-menopausal woman by administration of the dosage form for 23 to 26 days, beginning at day one of the menstrual cycle, followed by 2 to 5 pill- free or blank pill days, for a total of 28 days in the administration cycle.

2. The use as claimed in Claim 1 in which the estrogen is 17β-estradiol.

3. The use as claimed in Claim 1 or 2 in which the progestogen is levonorgestrel.

4. The use as claimed in Claim 1 or 2 in which the progestogen is gestodene, desogestrel or 3-ketodesogestrel.

5. The use as claimed in Claim 4 in which the progestogen is gestodene.

6. The use as claimed in Claim 1 in which the estrogen is in a dose of 1 mg of 17β-estradiol or an equivalent dose of ethinyl estradiol or mestranol and the progestogen is in a dose of 0.050 mg of levonorgestrel or an equivalent dose of gestodene, desogestrel or 3-ketodesogestrel.

7. The use as claimed in Claim 1 in which the estrogen is in a dose of 1mg of 17β-estradiol or an equivalent dose of ethinyl estradiol or mestranol and the progestogen is in a dose of 0.075 mg of levonorgestrel or an equivalent dose of gestodene, desogestrel or 3-ketodesogestrel.

8. A pack for providing a hormonal replacement therapy and contraception for a pre-menopausal woman which pack comprises
(a) 23 to 26 dosage forms each comprising an estrogen selected from:
0.75-1.50 mg of 17β-estradiol,
0.012-0.025 mg of ethinyl estradiol, and
0.025-0.050 mg of mestranol ;
and a progestogen selected from
0.035-0.085 mg of levonorgestrel,
0.015-0.060 mg of gestodene,
0.035-0.085 mg of desogestrel,
0.035-0.085 mg of 3-ketodesogestrel, and
0.10-0.30 mg of norethindrone,
and
(b) 2 to 5 blank pills or other indications to indicate that the daily administration of the 23 to 26 dosage forms should be followed by 2 to 5 pill-free or blank pill days, for a total of 28 days in the administration cycle.

9. A pack as claimed in Claim 8 in which the selected estrogen is 17β-estradiol.

10. A pack as claimed in Claim 8 or 9 in which the selected progestogen is levonorgestrel.

11. A pack as claimed in Claim 8 in which the progestogen is gestodene.

12. A pack as claimed in Claim 8 in which the estrogen is in a dose of 1mg of 17β- estradiol or an equivalent dose of ethinyl estradiol or mestranol and the progestogen is in a dose of 0.050 mg of levonorgestrel or an equivalent dose of gestodene, desogestrel or 3-ketodesogestrel.

13. A pack as claimed in Claim 8 in which the estrogen is in a dose of 1 mg of 17β-estradiol or an equivalent dose of ethinyl estradiol or mestranol and the progestogen is in a dose of 0.075 mg of levonorgestrel or an equivalent dose of gestodene, desogestrel or 3-ketodesogestrel.

14. A pack as claimed in any one of Claims 8 to 13 which comprises 24 dosage forms and 4 blank pills or other indications to indicate that no dosage form or a blank pill is administered for the last 4 days of the menstrual cycle.

15. A pack as claimed in any one of claims 8 to 13 which comprises 23 dosage forms and 5 blank pills or other indications to indicate that no dosage form or a blank pill is administered for the last 5 days of the menstrual cycle.

16. A pack according to Claim 8 comprising 24 dosage forms each comprising 1 mg of 17β-estradiol and 0.050 mg or 0.075 mg of levonorgestrel and 4 blank pills or other indications to indicate that no dosage form or a blank pill is administered for the last 4 days of the menstrual cycle.

17. A pack according to Claim 8 comprising 23 dosage forms each comprising 1 mg of 17β-estradiol and 0.50 mg or 0.075 mg of levonorgestrel and 5 blank pills or other indications to indicate that no dosage form or a blank pill is administered for the last 5 days of the menstrual cycle.

## Claims (Claims for the following Contracting State(s): AT, ES, GR)

1. The use of a composition comprising an estrogen selected from
0.75-1.50 mg of 17β-estradiol,
0.012-0.025 mg of ethinyl estradiol, and
0.025-0.050 mg of mestranol ;
and a progestogen selected from :
0.035-0.085 mg of levonorgestrel,
0.015-0.060 mg of gestodene,
0.035-0.085 mg of desogestrel,
0.035-0.085 mg of 3-ketodesogestrel, and
0.10-0.30 mg of norethindrone,
for the manufacture of a dosage form for providing hormonal replacement therapy and contraception for a pre-menopausal woman by administration of the dosage form for 23 to 26 days, beginning at day one of the menstrual cycle, followed by 2 to 5 pill-free or blank pill days, for a total of 28 days in the administration cycle.

2. The use as claimed in Claim 1 in which the estrogen is 17β-estradiol.

3. The use as claimed in Claim 1 or 2 in which the progestogen is levonorgestrel.

4. The use as claimed in Claim 1 or 2 in which the progestogen is gestodene, desogestrel or 3-ketodesogestrel.

5. The use as claimed in Claim 4 in which the progestogen is gestodene.

6. The use as claimed in Claim 1 in which the estrogen is in a dose of 1 mg of 17β-estradiol or an equivalent dose of ethinyl estradiol or mestranol and the progestogen is in a dose of 0.050 mg of levonorgestrel or an equivalent dose of gestodene, desogestrel or 3-ketodesogestrel.

7. The use as claimed in Claim 1 in which the estrogen is in a dose of 1 mg of 17β-estradiol or an equivalent dose of ethinyl estradiol or mestranol and the progestogen is in a dose of 0.075 mg of levonorgestrel or an equivalent dose of gestodene, desogestrel or 3-ketodesogestrel.

8. A process for preparing a pack for providing a hormonal replacement therapy and contraception for a pre-menopausal woman which process comprises associating
(a) 23 to 26 dosage forms each comprising an estrogen selected from:
0.75-1.50 mg of 17β-estradiol,
0.012-0.025 mg of ethinyl estradiol, and
0.025-0.050 mg of mestranol ;
and a progestogen selected from :
0.035-0.085 mg of levonorgestrel,
0.015-0.060 mg of gestodene,
0.035-0.085 mg of desogestrel,
0.035-0.085 mg of 3-ketodesogestrel, and
0.10-0.30 mg of norethindrone,
with
(b) 2 to 5 blank pills or other indications to indicate that the daily administration of the 23 to 26 dosage forms should be followed by 2 to 5 pill free or blank pill days, for a total of 28 days in the administration cycle.

9. A process as claimed in Claim 8 in which the selected estrogen is 17β-estradiol.

10. A process as claimed in Claim 8 or 9 in which the selected progestogen is levonorgestrel.

11. A process as claimed in Claim 8 in which the progestogen is gestodene.

12. A process as claimed in Claim 8 in which the estrogen is in a dose of 1 mg of 17β-estradiol or an equivalent dose of ethinyl estradiol or mestranol and the progestogen is in a dose of 0.050 mg of levonorgestrel or an equivalent dose of gestodene, desogestrel or 3-ketodesogestrel.

13. A process as claimed in Claim 8 in which the estrogen is in a dose of 1mg of 17β-estradiol or an equivalent dose of ethinyl estradiol or mestranol and the progestogen is in a dose of 0.075 mg of levonorgestrel or an equivalent dose of gestodene, desogestrel or 3-ketodesogestrel.

14. A pack as claimed in any one of Claims 8 to 13 which comprises 24 dosage forms and 4 blank pills or other indications to indicate that no dosage form or a blank pill is administered for the last 4 days of the menstrual cycle.

15. A process as claimed in any one of claims 8 to 13 which comprises 23 dosage forms and 5 blank pills or other indications to indicate that no dosage form or a blank pill is administered for the last 5 days of the menstrual cycle.

16. A process according to Claim 8 comprising 24 dosage forms each comprising 1 mg of 17β-estradiol and 0.050 mg or 0.075 mg of levonorgestrel and 4 blank pills or other indications to indicate that no dosage form or a blank pill is administered for the last 4 days of the menstrual cycle.

17. A process according to Claim 8 comprising 23 dosage forms each comprising 1 mg of 17β-estradiol and 0.050 mg or 0.075 mg of levonorgestrel and 5 blank pills or other indications to indicate that no dosage form or a blank pill is administered for the last 5 days of the menstrual cycle.

## Patentansprüche (Patentansprüche für folgende(n) Vertragsstaat(en): DE, FR, IT, NL, SE, LI, CH, BE, LU)

1. Verwendung einer Zusammensetzung umfassend ein Östrogen ausgewählt aus
0,75 bis 1,50 mg 17β-Östradiol,
0,012 bis 0,025 mg Ethinylöstradiol und
0,025 bis 0,050 mg Mestranol;
und ein Progestogen ausgewählt aus
0,035 bis 0,085 mg Levonorgestrel,
0,015 bis 0,060 mg Gestoden,
0,035 bis 0,085 mg Desogestrel,
0,035 bis 0,085 mg 3-Ketodesogestrel, und
0,10 bis 0,30 mg Norethindron
zur Herstellung einer Dosierungsform zum Vorsehen von Hormonersatztherapie und Empfängnisverhütung für eine prämenopauselle Frau durch Verabreichung der Dosierungsform während 23 bis 26 Tagen, beginnend am Tag eins des Menstruationszyklus, gefolgt von 2 bis 5 pillenfreien oder Blindpillentagen, während insgesamt 28 Tagen im Verabreichungszyklus.

2. Verwendung nach Anspruch 1, wobei das Östrogen 17β-Östradiol ist.

3. Verwendung nach Anspruch 1 oder 2, wobei das Progestogen Levonorgestrel ist.

4. Verwendung nach Anspruch 1 oder 2, wobei das Progestogen Gestoden, Desogestrel oder 3-Ketodesogestrel ist.

5. Verwendung nach Anspruch 4, wobei das Progestogen Gestoden ist.

6. Verwendung nach Anspruch 1, wobei das Östrogen in einer Dosis von 1 mg 17β-Östradiol oder einer äquivalenten Dosis von Ethinylöstradiol oder Mestranol vorliegt und das Progestogen in einer Dosis von 0,050 mg Levonorgestrel oder einer äquivalenten Dosis von Gestoden, Desogestrel oder 3-Ketodesogestrel vorliegt.

7. Verwendung nach Anspruch 1, wobei das Östrogen in einer Dosis von 1 mg 17β-Östradiol oder einer äquivalenten Dosis von Ethinylöstradiol oder Mestranol vorliegt und das Progestogen in einer Dosis von 0,075 mg Levonorgestrel oder einer äquivalenten Dosis von Gestoden, Desogestrel oder 3-Ketodesogestrel vorliegt.

8. Packung zum Vorsehen von Hormonersatztherapie und Empfängnisverhütung für eine prämenopauselle Frau, welche Packung:
(a) 23 bis 26 Dosierungsformen, jeweils umfassend ein Östrogen ausgewählt aus
0,75 bis 1,50 mg 17β-Östradiol,
0,012 bis 0,025 mg Ethinylöstradiol und
0,025 bis 0,050 mg Mestranol;
und ein Progestogen ausgewählt aus
0,035 bis 0,085 mg Levonorgestrel,
0,015 bis 0,060 mg Gestoden,
0,035 bis 0,085 mg Desogestrel,
0,035 bis 0,085 mg 3-Ketodesogestrel, und
0,10 bis 0,30 mg Norethindron
und
(b) 2 bis 5 Blindpillen oder andere Indikationen, um anzuzeigen, dass während insgesamt 28 Tagen im Verabreichungszyklus die tägliche Verabreichung der 23 bis 26 Dosierungsformen von 2 bis 5 pillenfreien oder Blindpillentagen gefolgt sein sollte,
umfasst.

9. Packung nach Anspruch 8, wobei das ausgewählte Östrogen 17β-Östradiol ist.

10. Packung nach Anspruch 8 oder 9, wobei das ausgewählte Progestogen Levonorgestrel ist.

11. Packung nach Anspruch 8, wobei das Progestogen Gestoden ist.

12. Packung nach Anspruch 8, wobei das Östrogen in einer Dosis von 1 mg 17β-Östradiol oder einer äquivalenten Dosis von Ethinylöstradiol oder Mestranol vorliegt und das Progestogen in einer Dosis von 0,050 mg Levonorgestrel oder einer äquivalenten Dosis von Gestoden, Desogestrel oder 3-Ketodesogestrel vorliegt.

13. Packung nach Anspruch 8, wobei das Östrogen in einer Dosis von 1 mg 17β-Östradiol oder einer äquivalenten Dosis von Ethinylöstradiol oder Mestranol vorliegt und das Progestogen in einer Dosis von 0,075 mg Levonorgestrel oder einer äquivalenten Dosis von Gestoden, Desogestrel oder 3-Ketodesogestrel vorliegt.

14. Packung nach einem der Ansprüche 8 bis 13, welche 24 Dosierungsformen und 4 Blindpillen oder andere Indikationen, um anzuzeigen, dass keine Dosierungsform oder eine Blindpille während der letzten 4 Tage des Menstruationszyklus verabreicht wird, umfasst.

15. Packung nach einem der Ansprüche 8 bis 13, welche 23 Dosierungsformen und 5 Blindpillen oder andere Indikationen, um anzuzeigen, dass keine Dosierungsform oder eine Blindpille während der letzten 5 Tage des Menstruationszyklus verabreicht wird, umfasst.

16. Packung nach Anspruch 8, umfassend 24 Dosierungsformen, jeweils umfassend 1 mg 17β-Östradiol und 0,050 mg oder 0,075 mg Levonorgestrel und 4 Blindpillen oder andere Indikationen, um anzuzeigen, dass keine Dosierungsform oder eine Blindpille während der letzten 4 Tage des Menstruationszyklus verabreicht wird.

17. Packung nach Anspruch 8, umfassend 23 Dosierungsformen, jeweils umfassend 1 mg 17β-Östradiol und 0,050 mg oder 0,075 mg Levonorgestrel und 5 Blindpillen oder andere Indikationen, um anzuzeigen, dass keine Dosierungsform oder eine Blindpille während der letzten 5 Tage des Menstruationszyklus verabreicht wird.

## Patentansprüche (Patentansprüche für folgende(n) Vertragsstaat(en): AT, ES, GR)

1. Verwendung einer Zusammensetzung umfassend ein Östrogen ausgewählt aus
0,75 bis 1,50 mg 17β-Östradiol,
0,012 bis 0,025 mg Ethinylöstradiol und
0,025 bis 0,050 mg Mestranol;
und ein Progestogen ausgewählt aus
0,035 bis 0,085 mg Levonorgestrel,
0,015 bis 0,060 mg Gestoden,
0,035 bis 0,085 mg Desogestrel,
0,035 bis 0,085 mg 3-Ketodesogestrel, und
0,10 bis 0,30 mg Norethindron
zur Herstellung einer Dosierungsform zum Vorsehen von Hormonersatztherapie und Empfängnisverhütung für eine prämenopauselle Frau durch Verabreichung der Dosierungsform während 23 bis 26 Tagen, beginnend am Tag eins des Menstruationszyklus, gefolgt von 2 bis 5 pillenfreien oder Blindpillentagen, während insgesamt 28 Tagen im Verabreichungszyklus.

2. Verwendung nach Anspruch 1, wobei das Östrogen 17β-Östradiol ist.

3. Verwendung nach Anspruch 1 oder 2, wobei das Progestogen Levonorgestrel ist.

4. Verwendung nach Anspruch 1 oder 2, wobei das Progestogen Gestoden, Desogestrel oder 3-Ketodesogestrel ist.

5. Verwendung nach Anspruch 4, wobei das Progestogen Gestoden ist.

6. Verwendung nach Anspruch 1, wobei das Östrogen in einer Dosis von 1 mg 17β-Östradiol oder einer äquivalenten Dosis von Ethinylöstradiol oder Mestranol vorliegt und das Progestogen in einer Dosis von 0,050 mg Levonorgestrel oder einer äquivalenten Dosis von Gestoden, Desogestrel oder 3-Ketodesogestrel vorliegt.

7. Verwendung nach Anspruch 1, wobei das Östrogen in einer Dosis von 1 mg 17β-Östradiol oder einer äquivalenten Dosis von Ethinylöstradiol oder Mestranol vorliegt und das Progestogen in einer Dosis von 0,075 mg Levonorgestrel oder einer äquivalenten Dosis von Gestoden, Desogestrel oder 3-Ketodesogestrel vorliegt.

8. Verfahren zur Herstellung einer Packung zum Vorsehen von Hormonersatztherapie und Empfängnisverhütung für eine prämenopauselle Frau, welches Verfahren das Vereinigen von
(a) 23 bis 26 Dosierungsformen, jeweils umfassend ein Östrogen ausgewählt aus
0,75 bis 1,50 mg 17β-Östradiol,
0,012 bis 0,025 mg Ethinylöstradiol und
0,025 bis 0,050 mg Mestranol;
und ein Progestogen ausgewählt aus
0,035 bis 0,085 mg Levonorgestrel,
0,015 bis 0,060 mg Gestoden,
0,035 bis 0,085 mg Desogestrel,
0,035 bis 0,085 mg 3-Ketodesogestrel, und
0,10 bis 0,30 mg Norethindron
mit
(b) 2 bis 5 Blindpillen oder andere Indikationen, um anzuzeigen, dass während insgesamt 28 Tagen im Verabreichungszyklus die tägliche Verabreichung der 23 bis 26 Dosierungsformen von 2 bis 5 pillenfreien oder Blindpillentagen gefolgt sein sollte,
umfasst.

9. Verfahren nach Anspruch 8, wobei das ausgewählte Östrogen 17β-Östradiol ist.

10. Verfahren nach Anspruch 8 oder 9, wobei das ausgewählte Progestogen Levonorgestrel ist.

11. Verfahren nach Anspruch 8, wobei das Progestogen Gestoden ist.

12. Verfahren nach Anspruch 8, wobei das Östrogen in einer Dosis von 1 mg 17β-Östradiol oder einer äquivalenten Dosis von Ethinylöstradiol oder Mestranol vorliegt und das Progestogen in einer Dosis von 0,050 mg Levonorgestrel oder einer äquivalenten Dosis von Gestoden, Desogestrel oder 3-Ketodesogestrel vorliegt.

13. Verfahren nach Anspruch 8, wobei das Östrogen in einer Dosis von 1 mg 17β-Östradiol oder einer äquivalenten Dosis von Ethinylöstradiol oder Mestranol vorliegt und das Progestogen in einer Dosis von 0,075 mg Levonorgestrel oder einer äquivalenten Dosis von Gestoden, Desogestrel oder 3-Ketodesogestrel vorliegt.

14. Verfahren nach einem der Ansprüche 8 bis 13, welches 24 Dosierungsformen und 4 Blindpillen oder andere Indikationen, um anzuzeigen, dass keine Dosierungsform oder eine Blindpille während der letzten 4 Tage des Menstruationszyklus verabreicht wird, umfasst.

15. Verfahren nach einem der Ansprüche 8 bis 13, welches 23 Dosierungsformen und 5 Blindpillen oder andere Indikationen, um anzuzeigen, dass keine Dosierungsform oder eine Blindpille während der letzten 5 Tage des Menstruationszyklus verabreicht wird, umfasst.

16. Verfahren nach Anspruch 8, umfassend 24 Dosierungsformen, jeweils umfassend 1 mg 17β-Östradiol und 0,050 mg oder 0,075 mg Levonorgestrel und 4 Blindpillen oder andere Indikationen, um anzuzeigen, dass keine Dosierungsform oder eine Blindpille während der letzten 4 Tage des Menstruationszyklus verabreicht wird.

17. Verfahren nach Anspruch 8, umfassend 23 Dosierungsformen, jeweils umfassend 1 mg 17β-Östradiol und 0,050 mg oder 0,075 mg Levonorgestrel und 5 Blindpillen oder andere Indikationen, um anzuzeigen, dass keine Dosierungsform oder eine Blindpille während der letzten 5 Tage des Menstruationszyklus verabreicht wird.

## Revendications (Revendications pour l'(les) Etat(s) contractant(s) suivant(s): BE, CH, DE, FR, IT, LI, LU, NL, SE)

1. Utilisation d'une composition comprenant un oestrogène choisi parmi :
0,75 à 1,50 mg de 17β-oestradiol,
0,012 à 0,025 mg d'éthinylestradiol, et
0,025 à 0,050 mg de mestranol, et
et un progestogène choisi parmi :
0,035 à 0,085 mg de lévonorgestrel,
0,015 à 0,060 mg de gestodène,
0,035 à 0,085 mg de désogestrel,
0,035 à 0,085 mg de 3-cétodésogestrel, et
0,10 à 0,30 mg de noréthindrone,
pour la fabrication d'une forme dosée destinée à la thérapeutique de substitution hormonale et à la contraception chez la femme préménopausée par administration de la forme dosée pendant 23 à 26 jours commençant au jour 1 du cycle menstruel, suivie de 2 à 5 jours sans pilule ou avec pilule neutre, pour un total de 28 jours du cycle d'administration.

2. Utilisation suivant la revendication 1, dans laquelle l'oestrogène est le 17β-oestradiol.

3. Utilisation suivant la revendication 1 ou 2, dans laquelle le progestogène est le lévonorgestrel.

4. Utilisation suivant la revendication 1 ou 2, dans laquelle le progestogène est le gestodène, le désogestrel ou le 3-cétodésogestrel.

5. Utilisation suivant la revendication 4, dans laquelle le progestogène est le gestodène.

6. Utilisation suivant la revendication 1, dans laquelle l'oestrogène est présent en une dose de 1 mg de 17β-oestradiol ou en une dose équivalente d'éthinylestradiol ou de mestranol et le progestogène est présent en une dose de 0,050 mg de lévonorgestrel ou en une dose équivalente de gestodène, de désogestrel ou de 3-cétodésogestrel.

7. Utilisation suivant la revendication 1, dans laquelle l'oestrogène est présent en une dose de 1 mg de 17β-oestradiol ou en une dose équivalente d'éthinylestradiol ou de mestranol et le progestogène est présent en une dose de 0,075 mg de lévonorgestrel ou en une dose équivalente de gestodène, de désogestrel ou de 3-cétodésogestrel.

8. Conditionnement destiné à la thérapeutique de substitution hormonale et à la contraception chez la femme préménopausée, lequel conditionnement comprend :
(a) 23 à 26 formes dosées dont chacune comprend un oestrogène choisi parmi :
0,75 à 1,50 mg de 17β-oestradiol,
0,012 à 0,025 mg d'éthinylestradiol, et
0,025 à 0,050 mg de mestranol, et
un progestogène est choisi parmi :
0,035 à 0,085 mg de lévonorgestrel,
0,015 à 0,060 mg de gestodène,
0,035 à 0,085 mg de désogestrel,
0,035 à 0,085 mg de 3-cétodésogestrel, et
0,10 à 0,30 mg de noréthindrone,
(b) 2 à 5 pilules neutres ou autres indications pour informer que l'administration quotidienne des 23 à 26 formes dosées doit être suivie de 2 à 5 jours sans pilule ou avec pilule neutre, pour un total de 28 jours du cycle d'administration.

9. Conditionnement suivant la revendication 8, dans lequel l'oestrogène choisi est le 17β-oestradiol.

10. Conditionnement suivant la revendication 8 ou 9, dans lequel le progestogène choisi est le lévonorgestrel.

11. Conditionnement suivant la revendication 8, dans lequel le progestogène est le gestodène.

12. Conditionnement suivant la revendication 8, dans lequel l'oestrogène est contenu en une dose de 1 mg de 17β-oestradiol ou en une dose équivalente d'éthinylestradiol ou de mestranol et le progestogène est contenu en une dose de 0,050 mg de lévonorgestrel ou en une dose équivalente de gestodène, de désogestrel ou de 3-cétodésogestrel.

13. Conditionnement suivant la revendication 8, dans lequel l'oestrogène est contenu en une dose de 1 mg de 17β-oestradiol ou en une dose équivalente d'éthinylestradiol ou de mestranol et le progestogène est contenu en une dose de 0,075 mg de lévonorgestrel ou en une dose équivalente de gestodène, de désogestrel ou de 3-cétodésogestrel.

14. Conditionnement suivant l'une quelconque des revendications 8 à 13, qui comprend 24 formes dosées et 4 pilules neutres ou autres indications pour informer qu'aucune forme dosée n'est administrée ou qu'une pilule neutre est administrée pendant les 4 derniers jours du cycle menstruel.

15. Conditionnement suivant l'une quelconque des revendications 8 à 13, qui comprend 23 formes dosées et 5 pilules neutres ou autres indications pour informer qu'aucune forme dosée n'est administrée ou qu'une pilule neutre est administrée pendant les 5 derniers jours du cycle menstruel.

16. Conditionnement suivant la revendication 8, comprenant 24 formes dosées dont chacune comprend 1 mg de 17β-oestradiol et 0,050 mg ou 0,075 mg de lévonorgestrel et 4 pilules neutres ou autres indications pour informer qu'aucune forme dosée n'est administrée ou qu'une pilule neutre est administrée pendant les 4 derniers jours du cycle menstruel.

17. Conditionnement suivant la revendication 8, comprenant 23 formes dosées dont chacune comprend 1 mg de 17β-oestradiol et 0,050 mg ou 0,075 mg de lévonorgestrel et 5 pilules neutres ou autres indications pour informer qu'aucune forme dosée n'est administrée ou qu'une pilule neutre est administrée pendant les 5 derniers jours du cycle menstruel.

## Revendications (Revendications pour l'(les) Etat(s) contractant(s) suivant(s): AT, ES, GR)

1. Utilisation d'une composition comprenant un oestrogène choisi parmi :
0,75 à 1,50 mg de 17β-oestradiol,
0,012 à 0,025 mg d'éthinylestradiol, et
0,025 à 0,050 mg de mestranol, et
et un progestogène choisi parmi :
0,035 à 0,085 mg de lévonorgestrel,
0,015 à 0,060 mg de gestodène,
0,035 à 0,085 mg de désogestrel,
0,035 à 0,085 mg de 3-cétodésogestrel, et
0,10 à 0,30 mg de noréthindrone,
pour la fabrication d'une forme dosée destinée à la thérapeutique de substitution hormonale et à la contraception chez la femme préménopausée par administration de la forme dosée pendant 23 à 26 jours commençant au jour 1 du cycle menstruel, suivie de 2 à 5 jours sans pilule ou avec pilule neutre, pour un total de 28 jours du cycle d'administration.

2. Utilisation suivant la revendication 1, dans laquelle l'oestrogène est le 17β-oestradiol.

3. Utilisation suivant la revendication 1 ou 2, dans laquelle le progestogène est le lévonorgestrel.

4. Utilisation suivant la revendication 1 ou 2, dans laquelle le progestogène est le gestodène, le désogestrel ou le 3-cétodésogestrel.

5. Utilisation suivant la revendication 4, dans laquelle le progestogène est le gestodène.

6. Utilisation suivant la revendication 1, dans laquelle l'oestrogène est présent en une dose de 1 mg de 17β-oestradiol ou en une dose équivalente d'éthinylestradiol ou de mestranol et le progestogène est présent en une dose de 0,050 mg de lévonorgestrel ou en une dose équivalente de gestodène, de désogestrel ou de 3-cétodésogestrel.

7. Utilisation suivant la revendication 1, dans laquelle l'oestrogène est présent en une dose de 1 mg de 17β-oestradiol ou en une dose équivalente d'éthinylestradiol ou de mestranol et le progestogène est présent en une dose de 0,075 mg de lévonorgestrel ou en une dose équivalente de gestodène, de désogestrel ou de 3-cétodésogestrel.

8. Procédé de fabrication d'un conditionnement destiné à la thérapeutique de substitution hormonale et à la contraception chez la femme préménopausée, lequel procédé comprend l'association :
(a) de 23 à 26 formes dosées dont chacune comprend un oestrogène choisi parmi :
0,75 à 1,50 mg de 17β-oestradiol,
0,012 à 0,025 mg d'éthinylestradiol, et
0,025 à 0,050 mg de mestranol, et
et un progestogène choisi parmi :
0,035 à 0,085 mg de lévonorgestrel,
0,015 à 0,060 mg de gestodène,
0,035 à 0,085 mg de désogestrel,
0,035 à 0,085 mg de 3-cétodésogestrel, et
0,10 à 0,30 mg de noréthindrone.
avec
(b) 2 à 5 pilules neutres ou autres indications pour informer que l'administration quotidienne des 23 à 26 formes dosées doit être suivie de 2 à 5 jours sans pilule ou avec pilule neutre, pour un total de 28 jours du cycle d'administration.

9. Procédé suivant la revendication 8, dans lequel l'oestrogène choisi est le 17β-oestradiol.

10. Procédé suivant la revendication 8 ou 9, dans lequel le progestogène choisi est le lévonorgestrel.

11. Procédé suivant la revendication 8, dans lequel le progestogène est le gestodène.

12. Procédé suivant la revendication 8, dans lequel l'oestrogène est présent en une dose de 1 mg de 17β-oestradiol ou en une dose équivalente d'éthinylestradiol ou de mestranol et le progestogène est présent en une dose de 0,050 mg de lévonorgestrel ou en une dose équivalente de gestodène, de désogestrel ou de 3-cétodésogestrel.

13. Procédé suivant la revendication 8, dans lequel l'oestrogène est présent en une dose de 1 mg de 17β-oestradiol ou en une dose équivalente d'éthynyloestradiol ou de mestranol et le progestogène est présent en une dose de 0,075 mg de lévonorgestrel ou en une dose équivalente de gestodène, de désogestrel ou de 3-cétodésogestrel.

14. Procédé suivant l'une quelconque des revendications 8 à 13, qui comprend 24 formes dosées et 4 pilules neutres ou autres indications pour informer qu'aucune forme dosée n'est administrée ou qu'une pilule neutre est administrée pendant les 4 derniers jours du cycle menstruel.

15. Procédé suivant l'une quelconque des revendications 8 à 13, qui comprend 23 formes dosées et 5 pilules neutres ou autres indications pour informer qu'aucune forme dosée n'est administrée ou qu'une pilule neutre est administrée pendant les 5 derniers jours du cycle menstruel.

16. Procédé suivant la revendication 8, comprenant 24 formes dosées dont chacune comprend 1 mg de 17β-oestradiol et 0,050 mg ou 0,075 mg de lévonorgestrel et 4 pilules neutres ou autres indications pour informer qu'aucune forme dosée n'est administrée ou qu'une pilule neutre est administrée pendant les 4 derniers jours du cycle menstruel.

17. Procédé suivant la revendication 8, comprenant 23 formes dosées dont chacune comprend 1 mg de 17β-oestradiol et 0,050 mg ou 0,075 mg de lévonorgestrel et 5 pilules neutres ou autres indications pour informer qu'aucune forme dosée n'est administrée ou qu'une pilule neutre est administrée pendant les 5 derniers jours du cycle menstruel.
